# EUROPEAN PATENT APPLICATION

(11) **EP 4 501 922 A1**
(43) Date of publication of application: **05.02.2025**
(21) Application number: 22942440.3
(22) Date of filing: 10.11.2022
(51) Int. Cl.: C07D 401/12, A61K 9/08, A61K 31/4439, A61P 1/04

(54) **CRYSTAL FORM OF VONOPRAZAN PYROGLUTAMATE AND PREPARATION METHOD THEREFOR**

(30) Priority: 20.05.2022 CN 202210553395
(71) Applicant: Harway Pharma Co., Ltd, Dongying, Shandong 257000 (CN); Harway (Weifang) Pharmaceutical Technology Co., Ltd., Weifang, Shandong 262700 (CN)
(72) Inventor: SONG, Weiguo, Dongying, Shandong 257000 (CN); WANG, Xilong, Dongying, Shandong 257000 (CN); JIA, Zongqing, Dongying, Shandong 257000 (CN); LI, Fahui, Dongying, Shandong 257000 (CN); SONG, Chenggang, Dongying, Shandong 257000 (CN); XU, Mingjie, Dongying, Shandong 257000 (CN); QIAO, Yujing, Dongying, Shandong 257000 (CN)
(74) Representative: Chung, Hoi Kan
(86) International application number: PCT/CN2022/131030
(87) International publication number: WO 2023/221419

(57) **Abstract**

The present invention relates to a crystal form of Vonoprazan pyroglutamate and a preparation method therefor. The crystal form of Vonoprazan pyroglutamate obtained in the present invention not only has good solubility, but also has excellent storage stability. The *in vitro* activity experiments have shown that the inhibitory effect of the crystal form I of the present invention on H⁺K⁺-ATPase is equivalent to that of TAK-438. The animal experiments have shown that the crystal form of the present invention can significantly inhibit histamine-induced secretion of stomach acid in rats via intravenous administration, and can exert the efficacy faster than the duodenal administration of TAK-438.

## Description

### Technical Field

The present invention relates to the field of crystal forms of drugs, and more particularly to a crystal form of Vonoprazan pyroglutamate and a preparation method therefor.

### Background Technology

Vonoprazan, chemically 5- (2-fluorophenyl) -N-methyl-1- (3-pyridylsulfonyl) -1H-pyrrole-3-methylamine, has the following chemical structural formula:

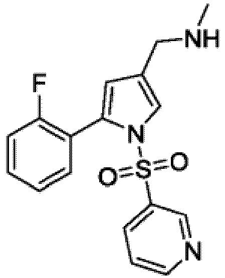

Vonoprazan fumarate (TAK-438), developed by Takeda in Japan and approved for marketing in Japan on 26 December 2014, is a new class of inhibitors of potassium-competitive acid blocker (P-CAB). P-CABs are lipophilic, weakly basic, have high dissociation constants, and are stable at low pH, thus Vonoprazan fumarate has a rapid-acting, robust, and long-lasting inhibitory effect on the gastric acid secretion. At the same time, this compound also has a premature termination effect on the gastric acid secretion by inhibiting the binding of K⁺ to H⁺-K⁺-ATPase (proton pump) in the final step of gastric acid secretion in gastric parietal cells, but its water solubility is poor. The crystal form and *in vitro* and *in vivo* biological activity of Vonoprazan pyroglutamate with good water solubility are studied, and a crystal form I of Vonoprazan pyroglutamate with good solubility, stability and *in vivo* biological activity is obtained.

### Summary of the Invention

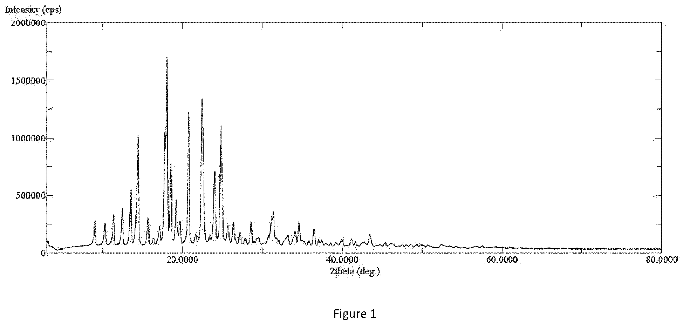

The present invention provides a crystal form I of a Vonoprazan pyroglutamate, wherein characteristic peaks are observed at 9.000 ± 0.200, 10.280 ± 0.200, 11.340 ± 0.200, 12.440 ± 0.200, 13.480 ± 0.200, 14.360 ± 0.200, 15.640 ± 0.200, 17.100 ± 0.200, 18.000 ± 0.200, 18.500 ± 0.200, 19.240 ± 0.200, 19.720 ± 0.200, 20.820 ± 0.200, 21.660 ± 0.200, 22.500 ± 0.200, 24.040 ± 0.200, 24.860 ± 0.200, 25.720 ± 0.200, 26.400 ± 0.200, 27.200 ± 0.200, 28.600 ± 0.200, 31.320 ± 0.200, 33.200 ± 0.200, 34.100 ± 0.220, 34.600 ± 0.200, 36.480 ± 0.200, and 43.460 ± 0.200 on X-ray powder diffraction patterns at 20 diffraction angles using Cu/Kα radiation. The crystal form I may also have characteristics essentially as represented by the X-ray powder diffraction pattern shown in Figure 1.

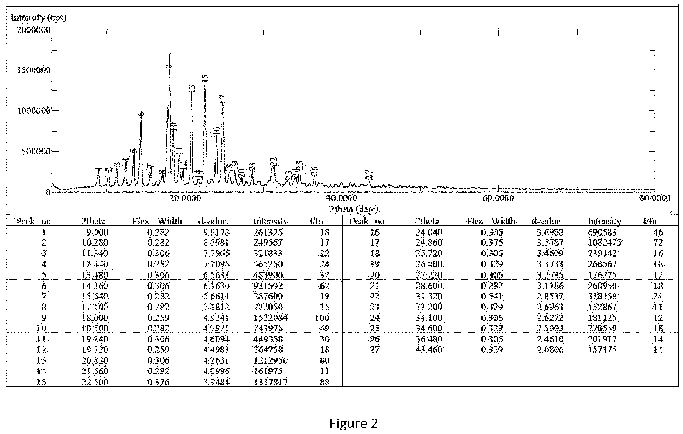

Another embodiment of the present invention provides a crystal form I of the Vonoprazan pyroglutamate salt, wherein an X-ray powder diffraction pattern with 27 characteristic peaks at 20 diffraction angles using Cu/Kα radiation is shown in Figure 2.

It should be appreciated by those skilled in the art that the relative intensity of peak heights of the characteristic 2θ peaks in the XRPD pattern, when measured for essentially the same crystal form, may change for a variety of reasons.

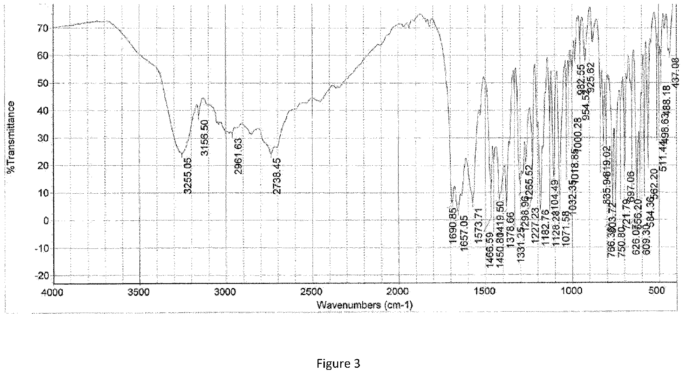

An infrared spectrum of the crystal form I of Vonoprazan pyroglutamate according to the present invention shows characteristic absorption peaks at positions of approximately 3255 ± 5, 2738 ± 5, 1690 ± 5, 1657 ± 5, 1573 ± 5 cm⁻¹. The crystal form I may also have characteristics essentially as represented by an infrared spectrum shown in Figure 3.

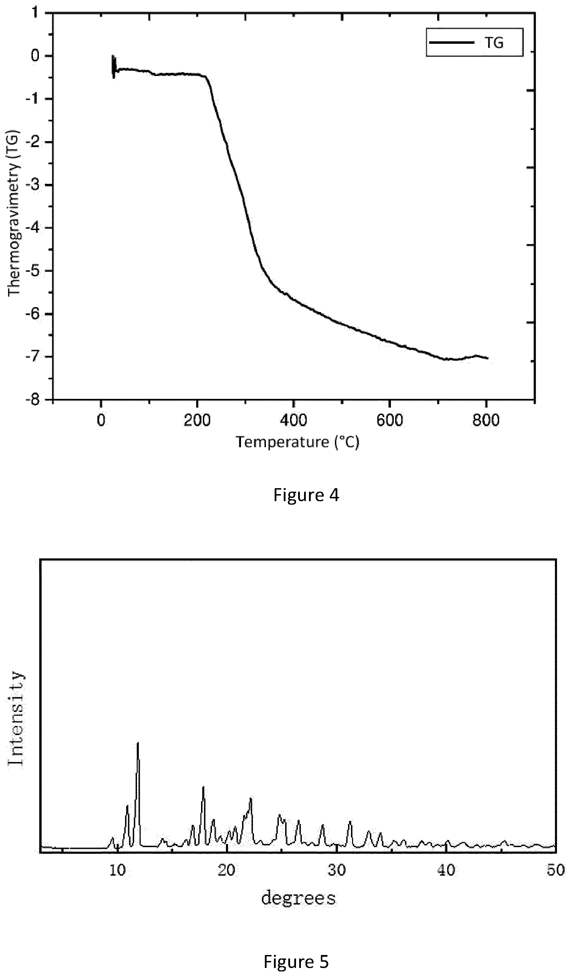

A thermogravimetric analysis of the crystal form I of Vonoprazan pyroglutamate provided by the present invention shows that the crystal form I loses weight for the first time at 297.9 ± 1.0°C by a thermogravimetric analysis, with a weight loss rate of 50.3±2.0%, which is caused by the decomposition of the substance. The TGA graph of the crystal form I is basically shown in Figure 4.

Another embodiment of the present invention provides a method for preparing the crystal form I of Vonoprazan pyroglutamate, which comprises the following steps: dissolving 5-(2-fluorophenyl) -N-methyl-1- (3-pyridylsulfonyl) -1H-pyrrole-3-methylamine pyroglutamate in methanol, dropping ketone solvent, stirring to crystallize, filtering, and drying to obtain the crystal form I.

The methanol dissolution can be carried out optionally at room temperature or at 20-35°C through artificial heating. As for ketone solvents, acetone, methyl ethyl ketone, etc. are preferred. The crystal precipitation process can be carried out at room temperature or at 0-5°C through artificial cooling. For the amount of methanol, ketone solvent, temperature and other parameters, the person skilled in the art can make a reasonable choice according to the actual situation of recrystallization (e.g. experimental phenomena).

Another embodiment of the present invention provides an application of the crystal form I of Vonoprazan pyroglutamate or pharmaceutically acceptable salts thereof in the preparation of a potassium-competitive acid blocker.

Another embodiment of the present invention provides an application of a pharmaceutical composition in the treatment and/or prevention of diseases, one or more of gastric ulcers, duodenal ulcers, reflux oesophagitis, erosive oesophagitis, gastro-oesophageal reflux diseases, Helicobacter infections and peptic ulcers, with the pharmaceutical composition using the crystal form I of Vonoprazan pyroglutamate or a pharmaceutically acceptable salt thereof as an active ingredient. The pharmaceutical composition may also comprise other potassium-competitive acid blockers or drugs inhibiting the gastric acid secretion. The pharmaceutical composition may also comprise pharmaceutically acceptable excipients (e.g., pharmaceutically acceptable carriers, diluents, or excipients, including solubilizers, surfactants, film formers, antioxidants, stabilizers, adhesives, lubricants, etc.). The pharmaceutical composition may be formulated as solid, liquid or semi-solid formulations, with tablets, capsules, injections (containing powder for injection), microemulsions, submicroemulsions, including extended-release tablets, extended-release capsules, and extended-release injections as the preferred dosage forms.

The term "pharmaceutically acceptable salt" in the present invention refers to non-toxic inorganic or organic acid addition salts and/or base addition salts, as described in Salt selection for basic drugs, Int. J. Pharm. (1986), 33, 201-217*.*

The pharmaceutical compositions of the present invention involve various dosage forms which can be prepared according to technical specifications and requirements in the pharmaceutical field (e.g., General Rules and Specifications for Preparations in the Chinese Pharmacopoeia 2015*),* textbooks or other existing techniques, as preparations meeting the clinical therapeutic and/or preventive needs for inhibiting the gastric acid secretion and various strengths and dosage forms suitable for such preparations (including solid preparations, liquid preparations and semi-solid preparations) such as sustained-release, controlled-release and enteric-coated capsules, tablets, microemulsions, submicroemulsions or injections containing the same single and multi-component combinations.

The X-ray powder diffraction (XRPD) analysis of the crystal form I of Vonoprazan pyroglutamate of the present invention is performed at ambient temperature and ambient humidity by Japanese Rigaku miniFlex600 powder X-ray diffractometer (3-80°, step: 0.0200°, speed: 10°/min), using Cu/Kα source (40kV, 40 mA). The infrared spectrum analysis of the present invention is completed by an American Thermo Nicolet 6700 infrared spectrophotometer under ambient temperature and ambient humidity using a KBr disk method. The thermogravimetric analysis (TGA) of the present invention is determined by a Swiss Mettler TGA/DSC1/1100LF simultaneous thermal analyzer at ambient temperature and ambient humidity, with a scanning rate of 10.00°C/min, maximum temperature of 800.00°C and protective gas of nitrogen. "Ambient temperature" generally refers to 0-40°C; "ambient humidity" generally refers to 30-80 % relative humidity.

The representative X-ray powder diffraction patterns, infrared spectra and thermogravimetric analyses of the crystal form I of Vonoprazan pyroglutamate described herein are illustrated in the accompanying drawings of the specification. The "representative X-ray powder diffraction pattern or infrared spectrum" refers to that the characteristics of the X-ray powder diffraction or infrared signatures of the crystal form basically conform to the overall morphology shown in the spectrum. It is understandable that during the test process, due to the influence of multiple factors (such as particle size of test samples, processing method of test samples, instruments, test parameters, test operation, etc.), there will be some differences in the peak position or peak intensity in the X-ray powder diffraction pattern or infrared spectrum measured in the same crystal form. Due to the differences between machines or between samples, the 2θ value of the X-ray powder diffraction pattern may vary slightly, and the experimental error of the 2θ value is generally ±0.2°; the experimental error of the absorption peaks in the infrared spectrum is generally ±5 cm⁻¹; the experimental error of the weight-loss temperature in the thermogravimetric analysis is generally ±1°C, and the experimental error of the weight-loss percentage is generally ±2.0%. The term "Vonoprazan pyroglutamate" in the present invention refers to one molecule of Vonoprazan corresponding to one molecule of L-pyroglutamic acid, i.e., a molar ratio of 5-(2-fluorophenyl) -N-methyl-1- (3-pyridylsulfonyl) -1H-pyrrole-3-methylamine to L-pyroglutamic acid of 1:1 in Vonoprazan pyroglutamate. The Vonoprazan pyroglutamate can be purchased commercially or prepared from 5- (2-fluorophenyl) -N-methyl-1-(3-pyridylsulfonyl) -1H-pyrrole-3-methylamine and L-pyroglutamic acid according to a method in the prior art (e.g., Chinese Patent Application No.: 201410154778.8). The Vonoprazan Fumarate, 5- (2-fluorophenyl) -N-methyl-1- (3-pyridylsulfonyl) -1H-pyrrole-3-(TAK-438 for short), described in the present invention is purchased commercially, with a purity of 99.5%.

The chromatographic conditions for HPLC purity testing of the product involved in the present invention are as follows:
Column: Ultimate XB-C18, column length: 150 mm, inner diameter: 4.6 mm, particle size: 5 µm
Flow rate: 1.2 mL/min; column temperature: 30°C; wavelength: 210 nm; injection volume: 20 µL; solvent: acetonitrile-water (volume ratio 5:95)
Mobile phase A: acetonitrile - 0.02 mol/L potassium dihydrogen phosphate (adjusted to pH 2.5 with phosphoric acid) (volume ratio 5:95)
Mobile phase B: methanol-acetonitrile-0.02 mol/L potassium dihydrogen phosphate (adjusted to pH 2.5 with phosphoric acid) (volume ratio 7:63:30)

The proportions of mobile phases A and B over time are as follows:

| Time (min) | 0 | 3 | 6 | 16 | 33 | 71 | 71.1 | 85 |
|---|---|---|---|---|---|---|---|---|
| A | 100% | 100% | 77% | 77% | 74% | 0% | 100% | 100% |
| B | 0% | 0% | 23% | 23% | 26% | 100% | 0% | 0% |

The advantages of the present invention over the prior art are as follows:
(1) A novel crystal form of Vonoprazan pyroglutamate, form I, obtained by the present invention not only has good solubility but also excellent storage stability; (2) The *in vitro* activity experiment showed that the crystal form I of the present invention showed comparable inhibition of H⁺K⁺-ATPase to TAK-438 and superior inhibition compared to Vonoprazan pyroglutamate (another crystal form, Figure 5) in the prior art (Chinese Patent Application No.: 201410154778.8); (3) The animal experiment showed that the intravenous administration of crystal form I of the present invention significantly inhibited histamine-induced gastric acid secretion in rats and exerted pharmacodynamic effects faster than the duodenal administration of TAK-438.

### Brief Description of the Drawings

Figure 1 is an X-ray powder diffraction pattern of a crystal form I of Vonoprazan pyroglutamate of the present invention;
Figure 2 shows 27 characteristic peaks of the X-ray powder diffraction of the crystal form I and specific parameters thereof such as diffraction angle 2θ, interplanar spacing d (Å),
relative intensity (%), etc.;
Figure 3 is an infrared spectrum of the crystal form I;
Figure 4 is a thermogravimetric analysis of the crystal form I;
Figure 5 is the X-ray powder diffraction pattern of product A;
Figure 6 is a graph of inhibition rate of microsomal H⁺K⁺-ATPase in porcine gastric mucosa by TAK438, product A and crystal form I;
Figure 7 is a comparison graph of body weights of rats in each group;
Figure 8 shows the effect of test articles in each group on histamine-induced gastric acid secretion;
Figure 9 shows the effect of test articles in each group on the inhibition rate of histamine-induced gastric acid secretion in rats.

### Specific Embodiments

The following detailed description of embodiments is given for clearness of understanding only and is in no way intended to limit the scope of the present invention or the implementation principles.

### Embodiment 1

5-(2-fluorophenyl)-N-methyl-1-(3-pyridylsulfonyl)-1H-pyrrole-3-methylamine pyroglutamate (10.0 g; HPLC purity: 98.0%; Vonoprazan pyroglutamate, hereafter referred to as product A, whose X-ray powder diffraction pattern is shown in Figure 5) is obtained according to the method documented in Embodiment 1 of Chinese Patent Application No.: 201410154778.8. A sample of 5-(2-fluorophenyl)-N-methyl-1-(3-pyridylsulfony)-1H-pyrrole -3-methyla-mine pyroglutamate was weighed 2.00 g and added to a 100 mL flask. Methanol (3.0 mL) was added and dissolved by stirring at 20-25°C for 10 minutes. Acetone (about 10.0 mL) was dropped at 20-25°C, with solid precipitates appeared during the dropping process, stirred for 30 minutes, cooled to 0-5°C, and stirred for another 1 hour. Subsequent filtration was carried out, followed by drying under vacuum at 50°C to generate 1.54 g of white solid as the crystal form I of Vonoprazan pyroglutamate (hereafter referred to as the crystal form 1). The X-ray powder diffraction pattern is shown in Figure 1, the specific diffraction angle 20, interplanar spacing d (Å), and relative intensity (%) are shown in Figure 2, the infrared pattern is shown in Figure 3, and the thermogravimetric analysis is shown in Figure 4, with HPLC purity of 99.8%.

### Embodiment 2 Stability test

(1) Deliquescence test: The product A, crystal form I, and TAK-438 were stored at 40°C and 75% relative humidity and observed for deliquescence for 30 days, with results recorded every 10 days as detailed in the table below.

| Test sample | Day 10 | Day 20 | Day 30 |
|---|---|---|---|
| Product A | Slight deliquescence | Obvious deliquescence | Obvious deliquescence |
| Crystal form I | Absence of deliquescence | Slight deliquescence | Obvious deliquescence |
| TAK-438 | Absence of deliquescence | Absence of deliquescence | Absence of deliquescence |

| | | | |
|---|---|---|---|
| "Absence of deliquescence" refers to that no moisture absorption is observed on the sample surface, "slight deliquescence" refers to that moisture absorption is observed on the sample surface, and "obvious deliquescence" refers to that lubrication can be observed on the sample surface. | | | |

(2) Thermal stability test: The product A and crystal form I were stored separately in open containers at 60°C and tested for storage stability after 30 days (Day 31). Samples at initial and after 30 days of storage were tested for purity by HPLC with three replicates per sample and averaged and the results are presented in the following table.

| Test sample | Product A | | Crystal form I | |
|---|---|---|---|---|
| Test time | Initial point | Day 31 | Initial point | Day 31 |
| Purity (%) | 98.0 | 94.6 | 99.8 | 99.2 |

According to the deliquescence and thermal stability tests, the crystal form I of the present invention is stable and easy to store for a long time.

### Embodiment 3 Solubility test and melting point test

Basic method for solubility test: Appropriate amount of the sample to be tested was taken out and ground into a fine powder, placed in a suitable container. Appropriate amount of water was added and shaken vigorously every 5 minutes for 30 seconds at 37°C + 2°C to observe dissolution within 30 minutes. If the sample is not dissolved, the quantitative water is added, and the solution is observed for dissolution by vigorously shaking for 30 seconds every 5 minutes at 37°C ± 2°C. Solubility was calculated at 37°C + 2°C. The measurements were repeated three times and the average value was taken.

Test method of melting point: An appropriate amount of the sample to be tested is taken out and finely powdered, and loaded into a capillary tube at one end that is melt-sealed to tightly aggregate the powder at the melt-sealed end of the capillary tube. The height of the loaded sample is about 3 mm and the heating rate of the melting point tester is 1°C/min. The assay was repeated three times and averaged and the results are presented in the following table.

| Test sample | Solubility (mg/mL) | Melting point (°C) |
|---|---|---|
| Product A | 1018 | 174-175 |
| Crystal form I | 901 | 167-168 |

Embodiment 4 Inhibition of H⁺K⁺-ATPase in microsomal vesicles of porcine gastric mucosa Test method: H⁺K⁺-ATPase is an ATPase localized in gastric mucosal parietal cells and activated exclusively by K⁺. In this experiment, H⁺K⁺-ATPase was extracted from the microsomal vesicles of porcine gastric mucosa, and ATPase could decompose ATP to generate ADP and inorganic phosphorus. The test article combined H⁺K⁺-ATPase and inhibited its activity. The level of ATPase activity was judged by measuring the amount of inorganic phosphorus, and the inhibitory effect of the test article was determined.

The specific method is as follows:
H⁺K⁺ATPase was extracted from porcine gastric mucosal microsomal vesicles by iPhase.

### (1) Quantification of BCA protein

Preparation of BCA working solution: An appropriate amount of BCA working solution was prepared by adding 50 volumes of BCA reagent A and 1 volume of BCA reagent B (50:1) according to the number of samples and mixed thoroughly. For example, 5.1 mL BCA working solution was prepared by adding 5 mL BCA reagent A to 100 µL BCA reagent B, mixing well. BCA working solutions are stable for 24 hours at room temperature.

### (2) Determination of protein concentration

1) Standard samples were added to the standard wells of a 96-well plate at 0, 1, 2, 4, 8, 12, 16, and 20 µL, and standard dilutions were added to make up to 20 µL, equivalent to standard sample concentrations of 0, 0.025, 0.05, 0.1, 0.2, 0.3, 0.4, 0.5 mg/mL, 1.25 mg/mL, and 2.5 mg/mL, respectively.
2) Samples were diluted at dilutions of 2×, 5×, and 10×, respectively, and 20 µL of sample was added to the sample wells of a 96-well plate. Sample volumes and dilution factors should be recorded.
3) 200 µL BCA working solution was added to each well and incubated at 37°C for 20-30 minutes.
   NOTE: The plate may also be left at room temperature for 2 hours or 60°C for 30 minutes. When the protein concentration is determined by BCA, the color will deepen with time, and the color reaction will accelerate due to the increase of temperature.
4) Absorbance of A562, or wavelengths between 540 and 595 nm, was measured with a microplate reader.
5) The protein concentration of the sample was calculated from the standard curve and the sample volume used.

### (3) Preparation of reaction system

### 1) Composition and preparation of enzyme reaction system

100mM TRIS-HCL (PH 6.8) : 100 µL 1M TRIS-HCL was added to 900 µL ddH₂O and mixed well. 37.5mM MgCl₂: 50 mg MgCL₂ was added to 14 µL ddH₂O and mixed well.

200mM KCI: 500 mg KCI was added to 33.5 mL ddH₂O and mixed well.

10 mM nigericin and valinomycin: 669 µL and 890 µL DMSO were added and mixed, dispensed into 1 µL nigericin and 5 µL valinomycin per tube, and stored at -20°C.

0.1 mg/mL microsomal vesicles: Protein concentrations were determined upon arrival and dispensed, stored at -80°C, and diluted with 5 mM TRIS-HCL for use.

### 2) Preparation of test article

Control compound: TAK438 was prepared as a 10 mM stock solution in DMSO and dispensed in 1 µL per tube.

Test compound: Product A and crystal form I were prepared as 10 mM stock solutions in ddH₂O, and dispensed in 1 µL per tube.

With 20 µM (or 40 µM) as the highest concentration, the control compound and the test compound were diluted stepwise 3-fold with DMSO and ddH₂O, respectively, to obtain the samples to be tested at 6 (or 7 or 8) concentration gradients.

### (4) Occurrence of reaction

### Enzymatic reaction

| | Test tube (µL) | Control tube (µL) |
|---|---|---|
| 100mM TRIS-HCL | 60 | 60 |
| 37.5mM MgCl₂ | 20 | 20 |
| 200mM KCI | 15 | / |
| ddH₂O | / | 15 |
| Nigericin | 1.5 | 1.5 |
| Valinomycin | 1.5 | 1.5 |
| 0.1g/mL microsomal vesicle | 50 | 50 |
| Compound | 1.5 | 1.5 |

Water bath at 37°C for 30 min, 16.5 µL ATP added and mixed well, and water bath at 37 ° C for 30 min

### (5) Color reaction

The chromogenic agent was prepared based on the instructions of *H⁺K⁺ ATP kit (Nanjing Jiancheng).

### (6) Plate reading

Per the enzymatic reaction volume of 150 µL, 45 µL of chromogen was added and mixed well, allowed to stand for 2 min, and the absorbance light signal at 660 nm was read by a microplate reader.

### (7) Data statistics

1) The inhibition rate was calculated according to the following formula: inhibition rate (%) =(1-(RLU_{compound}-RLU_{blank})/(RLU_{DMSO}-RLU_{blank}))×100%_{∘}
2) Graphpad PrismS was used to draw the pharmacodynamic inhibition rate curve and calculate the IC₅₀ value, with a 4-parameter model [fit= (A+((B-A)/(1+ ((C/x)^D)))] used.

### (8) Experimental results

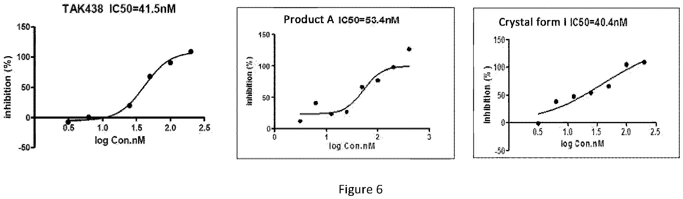

1) Quantification of BCA protein: The average protein concentration was calculated to be 3 mg/mL based on a BSA standard curve.
2) The inhibitory effects of TAK438, product A, and crystal form I on microsomal vesicle H⁺K⁺-ATPase in porcine gastric mucosa are shown in the following table and Figure 6.

| Test sample | IC₅₀ (nM) |
|---|---|
| TAK438 | 41.5 |
| Product A | 53.4 |
| Crystal form I | 40.4 |

Embodiment 5 Effect of intravenous injection on histamine-induced gastric acid secretion in rats

Surgical modeling: Rats were anesthetized with intraperitoneal injection of ethyl carbamate at a dose of 1.5 g/kg. Rats under complete anesthesia were shaved from the neck and abdomen, and were intubated to maintain airway patency during surgery. The cardiac perfusion tube was orally inserted about 1 cm into the stomach of rats, and the other end of the cardiac perfusion tube was connected to a constant flow pump. A small incision was made below the xiphoid cartilage to find the duodenum, an incision was made transversely, the end of the collection tube of gastric pyloric samples was passed along the duodenum into the stomach about 1 cm through the wound, the duodenum was tightened with the collection tube of gastric pyloric samples with a suture, and the other end of the duodenal incision was tightened. Gastric lavage remnants were rapidly flushed along the esophago-cardia cannula with 150 mL of 37°C prewarmed saline, outflowed through the collection tube of gastric pylorus samples, and the abdominal wall was sutured to the skin. Perfusion was continued with normal saline at a rate of 1 mL/min for 30 min, the other end of the col-lection tube for gastric pyloric samples was left *in vitro,* gastric juice samples were collected in conical flasks (one sample per 20 min of col-lection), and two basal gastric juice samples were collected, at which time basal gastric acid secretion reached a steady state. Histamine-induced gastric acid secretion: Histamine was administered to rats by continuous tail vein injection with a microinjection pump at a dose of 20 µmol/kg/hr. Gastric acid was collected after 80 min, and after three time points, histamine secretion reached a steady state in rats, which was grouped according to the value of gastric acid secretion after histamine administration in rats, and candidate drugs or vehicle controls were administered based on the protocol. Gastric acid was collected continuously, with gastric acid collected at 9 time points after administration, at which time the drug efficacy reached a steady state.

The details are as follows:

### (1) Test drug

Positive drug: Vonoprazan fumarate (TAK-438).

Preparation method: The whole preparation process was performed in the dark.

The required amount of Vonoprazan fumarate was weighed, added with an appropriate amount of 0.5% CMC-Na, stirred, sonicated to obtain a homogeneous suspension, and diluted sequentially to the corresponding concentration according to the concentration required by the protocol.

Test article: Crystal form I (expressed as HW-N2001 in the experiment).

Preparation method: The whole preparation process was performed in the dark.

The required amount of HW-N2001 was weighed, added with appropriate physiological saline, and mixed thoroughly to obtain a clear solution, and the pH was adjusted to a range of 3.2 to 3.8. Based on the concentrations required by the protocol, the corresponding concentrations were successively diluted.

### (2) Laboratory animal husbandry and instructions

### 1) Animal information

Breed and strain: SD rats.

Grade: SPF grade.

Sex: female, male.

Source: Shanghai Jihui Laboratory Animal Care Co., Ltd. (license No.: SCXK (Shanghai) 2017-0012).

Certificate No.: female: 20170012019182, 20170012019726; male: 20170012017891, 20170012018177, 20170012018680, 20170012018969, 20170012019726.

Number of animals: 130 animals were purchased, 118 animals (51 females and 67 males) in good health were selected for the experiment.

The age of animals at the beginning of the experiment: 6-9 weeks old, the weight of animals at the beginning of the experiment: 160-220 g for females and 180-260 g for males, and the period of adaptation to the environment: 5-7 days.

### 2) Housing environment

The animals were housed in SPF-grade animal room, in accordance with the standard temperature of 23±2°C, humidity of 40-70%, light condition of artificial illumination and 12-hour light/dark cycle specified in GB 14925-2010.

Type of bedding: corncob bedding (Dezhou Goodway Agriculture Science and Technology Co., Ltd., batch No.: GMCC202102170-2).

Type of feeds: SPF low-protein maintenance feeds for rat and mouse (Jiansu Xietong Pharmaceutical Bioengineering Co., Ltd., 20210225 (X)).

Feeding method: ad libitum.

Type of drinking water: autoclaved drinking water for test animals.

Water supply method: water bottles were used for free access.

### 3) Animal selection and fasting

Animals used for the experiment will remain in good health and will be fasted but not deprived of water for 24 hours prior to the experiment.

### 4) Ethics approval for animal use

This experimental project has been approved by the Experimental Animal Ethics Committee of Suzhou Institute of Drug Innovation, Shanghai Institute of Materia Medica, Chinese Academy of Sciences, with IACUC number of 2021-06-WZC-10.

### 5) Description of experimental animal selection

Common mammals, such as mice, rats, dogs and monkeys, were used as experimental animals in the experiment. These animals are commonly used for pharmacokinetic parameters, pharmacodynamics and toxicology studies. The number of animals used was the minimum required to judge the inter-individual variability of experimental animals. Animals used in the experiment must be healthy and acclimatized to the environment, with free access to food and water. In this experiment, SD rats of both sexes were used for the test.

### (3) Experimental design

### 1) Experimental grouping

One hundred and eighteen of 130 SD rats in good health were selected and randomly divided into 8 groups according to body weight and base-line value after histamine administration after the end of the adaptation period, as detailed in the table below.

| Group | Number of Animals | | Test Substance | Dosage | Volume | Administration Mode |
|---|---|---|---|---|---|---|
| | ♀ | ♂ | | mg/kg | mL/kg | |
| 1 | 4 | 3 | Naïve | NA | NA | NA |
| 2 | 8 | 15 | Vehicle | NA | 4 | i.v. |

### 2) Animal model preparation and experimental process

### Instrument and Reagent Preparation:

The normal saline was placed in the beaker and the beaker was placed in a water bath to maintain a water temperature of 37°C.

The flow rate (1 mL/min) of constant flow pump was adjusted for later use.

Histamine dihydrochloride: Histamine dihydrochloride (0.6 mg/mL in normal saline) was freshly prepared.

Ethyl carbamate: Appropriate amount of ethyl carbamate was weighed, added with normal saline, and dissolved by sonication to prepare a final concentration of 0.3 g/mL for later use. Phenolphthalein: Appropriate amount of phenolphthalein was weighed, and anhydrous ethanol was added to prepare a final concentration of 0.5% for later use.

Alkaline titrant: An appropriate amount of 0.1 mol/L NaOH solution was taken, added with fresh deionized water and diluted to 0.01 mol/L for later use.

Heparin sodium: An appropriate amount of heparin sodium was weighed, added with normal saline, and dissolved to a final concentration of 100 U/mL for later use.

### Animal preparation:

118 SD rats (male and female) were fasted but not deprived of water for 24 hours after acclimation and randomly divided into 8 groups (male and female).

### Preparation and fabrication of cannula:

Endotracheal tube: A medical polyethylene plastic tube (outer diameter 2 mm, inner diameter 1.5 mm) was taken approximately 2-3 cm for later use.

Gastric cardia perfusion tube: A medical polyethylene hose (outer diameter 2 mm, inner diameter 1.5 mm) was taken approximately 20 cm as a cardia gastric tube for later use. Collection tube of gastric pylorus sample: A medical polyethylene hose (outer diameter 4 mm, inner diameter 3 mm) was taken approximately 30 cm as the pylorus cannula end for later use.

Histamine perfusion tube: 10 mL histamine dihydrochloride is drawn by a 10 mL syringe, connected with an infusion extension tube, wrapped in tin paper to protect from light, and fixed in a syringe pump for later use.

Animal anesthesia and nursing: Rats were anesthetized by intraperitoneal injection of ethyl carbamate at a dose of 1.5 g/kg, and the hair on the neck and abdomen was shaved.

### Endotracheal cannula:

To keep the airway unobstructed during surgery, a 2-cm wound was longitudinally opened in the neck (at the epiglottic cartilage) to separate the muscles to expose the epiglottic cartilage from the trachea, and a segment of suture was passed from the trachea by separating the trachea. A "T" shaped incision was made over the trachea, and the endotracheal tube was gently inserted approximately 1 cm proximal to the endotracheal incision and tightened with sutures. The patency of the endotracheal tube was checked to suture the wound and the other end of the endotracheal tube was exposed outside the skin incision.

### Gastric cardia perfusion cannula:

The distance from the mouth to the stomach (about 15 cm) was measured and marked on the gastric cardia perfusion tube, and the gastric cardia perfusion tube was inserted transesophagically to the mark to ensure that the gastric cardia perfusion tube passed through the cardiac sphincter to reach the stomach. The gastric cardia perfusion tube is connected to a silicone tube on the constant flow pump.

### Cannula for gastric pyloric sample collection:

Below the xiphoid cartilage, a small opening was opened along the ventral white line to open the abdominal cavity to examine the position of the gastric cardia perfusion tube. An incision was made transversely on the duodenum 1 cm below the pylorus, the end of the collection tube of gastric pylorus samples was passed along the duodenum into the stomach about 1 cm through the wound, the duodenum was tied to the col-lection tube of gastric pylorus samples with a suture, and the other end of the duodenal incision was tied. Gastric lavage remnants were rapidly flushed along the esophago-cardia cannula with 150 mL of 37°C pre-warmed saline, out-flowed through the collection tube of gastric pylorus samples, and the abdominal wall was sutured to the skin. Perfusion was continued with normal saline at a rate of 1 mL/min for 30 min, the other end of the col-lection tube for gastric pyloric samples was left in vitro, gastric juice samples were collected in conical flasks (one sample per 20 min of collection), and two basal gastric juice samples were collected, at which time basal gastric acid secretion reached a steady state.

### Sample collection and processing:

Collected gastric juice samples were taken and titrated with 0.01 N sodium hydroxide solution by a basic burette to PH7 as the endpoint, with the phenolphthalein indicator as a color indicator. The titration volume was measured and the acid secretion is calculated according to the following formula: acid secretion (µEq) = volume of NaOH (mL) ×10⁻³×0.01 mol/L×10⁶.

Indwelling needle of tail vein: The tail vein is disinfected by alcohol, the indwelling needle is inserted into the tail vein, the indwelling needle core is pulled out, 0.5 mL of heparin sodium solution is pushed in, and the syringe is pulled out to screw the heparin sodium cap.

Two basal gastric juice samples were collected, and an infusion extension tube prematurely inhaled with histamine was connected to the indwelling needle of tail vein in rats after basal gastric acid levels were stabilized. The syringe pump was opened, and histamine was calculated according to the dose of 20 µmol/kg/hr and the intravenous drip rate of each animal in the syringe pump was set. Gastric acid samples after histamine administration were collected after 80 minutes of continuous injection of histamine solution. Gastric juice was collected after three doses of histamine, and when the gastric acid secretion reached a steady state, the test article was administered intravenously or the positive drug or vehicle control was administered intraduodenally based on groups. The collection of gastric juice was continued, with the base consumption reaching a steady state approximately 3 hours after administration, for a total of approximately 14 time points.

**Body weight:** Body weight was measured before fasting on the day before the experiment and before anesthesia on the day of the experiment.

**Total acidity of gastric juice:** The total acidity of gastric juice was deter-mined by the acid-base titration with 0.5% phenolphthalein as a color indicator, and the titration endpoint was pH 7.0.

### (4) Data statistics

Where not specifically noted, experimental data were presented as mean plus or minus standard deviation (Mean ± SD) and tested for normality using the One-Sample Kolmogorov-Smirnov Test. One-way analysis of variance (one way ANOVA) was used when data within groups met normal distribution. When the variance was homogeneous, multiple comparisons among the means of multiple samples were per-formed by LSD test, and P<0.05 was considered to be a significant difference; When the variances were heterogeneous, the Games-Howell test was used for multiple comparisons between the means of multiple samples. If the data within groups were not normally distributed, the independent-samples T test with non-parametric test was used, and P < 0.05 was considered to be significantly different among multiple samples, and all statistical analysis of data was performed using a statistical soft-ware of SPSS 16.0.

### (4) Experimental results

### 1) Comparison of body weight between groups

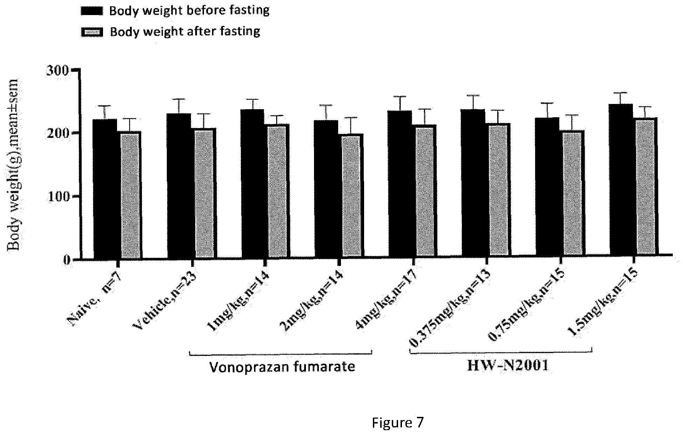

During the whole experiment, no abnormalities were observed in the state of animals in each group, and there was no significant difference in body weights before and after fasting (Figure 7).

### 2) Effect of test article on histamine-induced gastric acid secretion

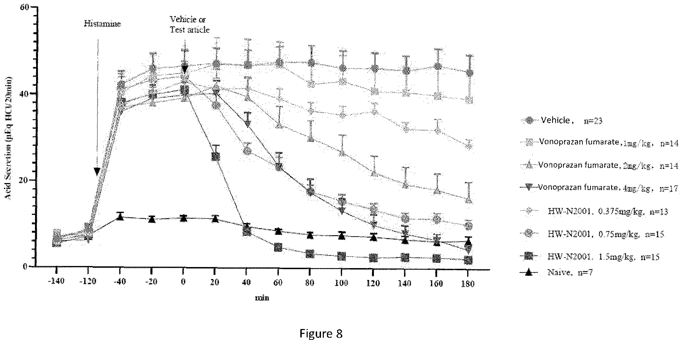

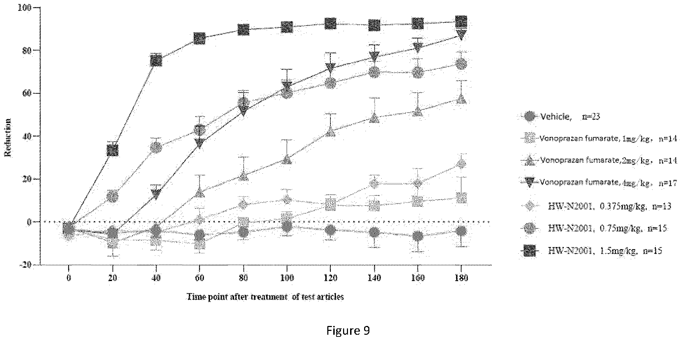

There was no significant difference in baseline values between SD rats in each group before the histamine administration, and after the histamine administration, there was a significant difference between the vehicle group and the naive group, whereas there was no significant difference in comparison with the positive drug (Vonoprazan fumarate) or the test article (HW-N2001) in each dose group. After the intraduodenal administration of positive drug or intravenous administration of test article, the gastric acid secretion and the inhibition rate of gastric acid secretion in each group were different from those in vehicle group, as shown below (Figures 8 and 9).

Vehicle group: The gastric acid was collected at two time points before the histamine administration in rats, and the baseline values of gastric acid secretion were 6.43 ± 2.37 and 8.83 ± 5.1 (µEq HCl/20 min), respectively, which were not significantly different from those in the naive group; The gastric acid secretion was significantly increased and reached the steady state at three time points (42.3 ± 14.58, 45.96 ± 16.61, 46.65 ± 16.965 µEq HCl/20 min) after 80 min of the histamine administration in rats, which were significantly different from those in the naive group; The vehicle group was significantly different from the naive group at each time point following the intravenous administration.

Vonoprazan fumarate (1 mg/kg) group: Gastric acid secretion reached the steady state after the histamine administration in animals, and was not significantly decreased in rats after administration, with no statistical difference compared with the vehicle group.

Vonoprazan fumarate (2 mg/kg) group: After the histamine administration, the gastric acid secretion reached the steady state, and gradually decreased from 60 min to 180 min after administration. At 60 min after administration, the gastric acid secretion was 33.21 ± 15.63 (µEq HCl/20 min) and the inhibition rate of gastric acid secretion was 13.71% ± 2.12, which were statistically different from the vehicle group; At 180 min after administration, the gastric acid secretion was 16.21 ± 14.46 (µEq HCl/20 min) and the inhibition rate of gastric acid secretion was 57.48% ± 2.27, which were significantly different from the blank control group.

Vonoprazan fumarate (4 mg/kg) group: After the histamine administration, gastric acid secretion in rats reached the steady state, and gradually decreased from 40 min to 180 min after administration. At 40 min after administration, the gastric acid secretion in rats was 33.18 ± 11.15 (µEq HCl/20 min), which was not statistically different from the vehicle group, and the inhibition rate of gastric acid secretion was 12.47% ± 1.13, which was statistically different from the vehicle group. At 180 min after administration, the gastric acid secretion was 4.41 ± 4.02 (µEq HCl/20 min), and the inhibition rate of gastric acid secretion was 87.03% ± 0.81, which was significantly different from the blank control group.

HW-N2001 (0.375 mg/kg) group: The steady state of gastric acid secretion was achieved in animals given histamine, and the gastric acid secretion in rats gradually decreased from 60 min to 180 min after administration. At 60 min after administration, the gastric acid secretion was 39.15 ± 8.54 (µEq HCl/20 min) and the inhibition rate of gastric acid secretion was 4.34% ± 1.28, which was not statistically different from vehicle group. At 180 min after administration, the gastric acid secretion was 28.46 ± 5.71 (µEq HCl/20 min), and the inhibition rate of gastric acid secretion was 27.52% ± 1.43, which was significantly different from the blank control group.

HW-N2001 (0.75 mg/kg) group: The steady state of gastric acid secretion was achieved in animals given histamine, and the gastric acid secretion in rats gradually decreased from 20 min to 180 min after administration. At 20 min after administration, the gastric acid secretion in rats was 37.53 ± 9.87 (µEq HCl/20 min), which was not significantly different from the blank control group, and the inhibition rate of gastric acid secretion was 11.6% ± 0.82, which was statistically different from the vehicle group. At 180 min after administration, the gastric acid secretion was 10 ± 5.32 (µEq HCl/20 min), and the inhibition rate of gastric acid secretion was 73.68% ± 1.47, which was significantly different from the blank control group. HW-N2001 (1.5 mg/kg) group: After the histamine administration, the gastric acid secretion reached a steady state, and from 20 min to 80 min after administration, the gastric acid secretion gradually decreased and the efficacy in rats reached a steady state. At 20 min after administration, the gastric acid secretion in rats was 25.67 ± 10.15 (µEq HCl), and the inhibition rate of gastric acid secretion was 33.28% ± 0, which were statistically different from the vehicle group. At 80 min after administration, the gastric acid secretion was 3.4 ± 1.18 (µEq HCl) and the inhibition rate of gastric acid secretion was 89.64% ± 0, which were significantly different from the blank control group. At 180 min after administration, the gastric acid secretion in rats was 2.2 ± 0.56 (µEq HCl), and the inhibition rate of gastric acid secretion was 93.56% ± 0.09, which were significantly different from the blank control group.

### (5) Conclusion

In the present invention, the pharmacodynamic effect of intravenous administration of the crystal form I (HW-N2001) of Vonoprazan pyroglutamate on the gastric acid secretion in SD rats was investigated by the continuous detection of a model of histamine-induced gastric acid secretion.

For the duodenal administration of Vonoprazan fumarate, the effect was not significant at 1 mg/kg, and the pharmacodynamic effect gradually exerted at 60 min and 140 min after administration and inhibited gastric acid secretion in rats at 2 and 4 mg/kg, respectively, with a certain dose-response relationship. However, the drug had a slow onset of action and reached the strongest efficacy at 180 min, with an ID₅₀ of 1.87 mg/kg.

For the intravenous administration of HW-N2001 (0.375 mg/kg, 0.75 mg/kg, 1.5 mg/kg), the gastric acid secretion in treatment groups was significantly lower than the vehicle group, with a certain dose-response relationship. In 0.75 and 1.5 mg/kg dose groups, the pharmacodynamic effect was gradually exerted at 20 min after administration, and the pharmacodynamic effect reached a stable state at 60-100 min after administration. The ID₅₀ was 0.53 mg/kg at 180 min after administration.

In conclusion, the intravenous administration of crystal form I (HW-N2001) of Vonoprazan pyroglutamate disclosed in the present invention significantly inhibited the histamine-induced gastric acid secretion in rats and exerted pharmacodynamic effects faster than the duodenal administration of Vonoprazan fumarate.

## Claims

1. A crystal form I of Vonoprazan pyroglutamate, wherein characteristic peaks are observed at 9.000 ± 0.200, 10.280 ± 0.200, 11.340 ± 0.200, 12.440 ± 0.200, 13.480 ± 0.200, 14.360 ± 0.200, 15.640 ± 0.200, 17.100 ± 0.200, 18.000 ± 0.200, 18.500 ± 0.200, 19.240 ± 0.200, 19.720 ± 0.200, 20.820 ± 0.200, 21.660 ± 0.200, 22.500 ± 0.200, 24.040 ± 0.200, 24.860 ± 0.200, 25.720 ± 0.200, 26.400 ± 0.200, 27.200 ± 0.200, 28.600 ± 0.200, 31.320 ± 0.200, 33.200 ± 0.200, 34.100 ± 0.220, 34.600 ± 0.200, 36.480 ± 0.200, and 43.460 ± 0.200 on X-ray powder diffraction patterns at 20 diffraction angles using Cu/Kα radiation.

2. A crystal form I of Vonoprazan pyroglutamate, wherein the X-ray powder diffraction pattern with 27 characteristic peaks at 20 diffraction angles using Cu/Kα radiation is shown in Figure 1 or Figure 2.

3. The crystal form I of Vonoprazan pyroglutamate according to any one of claims 1-2, wherein an infrared spectrum of the crystal form I shows characteristic absorption peaks at positions of approximately 3255 ± 5, 2738 ± 5, 1690 ± 5, 1657 ± 5, 1573 ± 5 cm⁻¹.

4. The crystal form I of Vonoprazan pyroglutamate according to any one of claims 1 to 3, wherein the crystal form I loses weight for the first time at 297.9 ± 1.0°C by a thermogravimetric analysis, with a weight loss rate of 50.3±2.0%.

5. A preparation method of the crystal form I of Vonoprazan pyroglutamate according to any one of claims 1 to 4, comprising: dissolving 5-(2-fluorophenyl)-N-methyl-1-(3-pyridylsulfonyl)-1H-pyrrole-3-methylamine pyroglutamate in methanol, dropping ketone solvent, stirring to crystallize, filtering, and drying to obtain crystal form I.

6. An application of the crystal form I of Vonoprazan pyroglutamate or a pharmaceutically acceptable salt thereof according to any one of claims 1 to 4 in the preparation of a potassium-competitive acid blocker.

7. An application of the crystal form I of Vonoprazan pyroglutamate or the pharmaceutically acceptable salt thereof according to any one of claims 1 to 4 in the preparation of drugs inhibiting gastric acid secretion.

8. The application according to claim 7, wherein the drug is used for treating and/or preventing diseases such as gastric ulcers, duodenal ulcers, reflux esophagitis, erosive esophagitis, gastroesophageal reflux disease, Helicobacter infections and peptic ulcers.

9. An application of a pharmaceutical composition in the treatment and/or prevention of diseases, one or more of the gastric ulcer, duodenal ulcer, reflux esophagitis, erosive esophagitis, gastroesophageal reflux disease, Helicobacter infection and peptic ulcer, with the pharmaceutical composition using the crystal form I of Vonoprazan pyroglutamate or a pharmaceutically acceptable salt thereof according to any one of claims 1 to 4 as an active ingredient.

10. The pharmaceutical composition according to claim 9, further comprising other potassium-competitive acid blockers or drugs inhibiting the gastric acid secretion.

11. The pharmaceutical composition according to any one of claims 9 to 10, further comprising pharmaceutically acceptable excipients, such as pharmaceutically acceptable carriers, diluents, or excipients, including solubilizers, surfactants, film formers, antioxidants, stabilizers, adhesives, lubricants, etc.

12. The pharmaceutical composition according to any one of claims 9 to 11, wherein the pharmaceutical composition may be formulated as solid, liquid or semi-solid formulations, with tablets, capsules, injections (containing powder for injection), microemulsions, submicroemulsions, including extended-release tablets, extended-release capsules, and extended-release injections as preferred dosage forms.
